# EUROPÄISCHE PATENTSCHRIFT

(11) **EP 2 604 296 B1**
(45) Veröffentlichungstag und Bekanntmachung des Hinweises auf die Patenterteilung: **14.05.2014**
(21) Anmeldenummer: 11401664.5
(22) Anmeldetag: 13.12.2011
(51) Int. Cl.: A61L 2/24, A61B 19/00, B08B 9/00, A47L 15/42

(54) **Spülautomat, insbesondere Desinfektionsautomat, Endoskopspüler**
Washing machine, in particular disinfection machine, endoscope washer
Automate de rinçage, notamment automate de désinfection, dispositif de rinçage d'endoscope

(43) Veröffentlichungstag der Anmeldung: 19.06.2013
(73) Patentinhaber: Miele & Cie. KG, 33332 Gütersloh (DE)
(72) Erfinder: Heitmann, Michael, 33739 Bielefeld (DE); Mayer, Josef, 5112 Lamprechtshausen (AT); Rüenbrink, Olaf, 32139 Spenge (DE)

(56) Entgegenhaltungen:
- EP-A2- 0 738 493
- WO-A2-02/11602
- DE-A1- 19 514 303

## Beschreibung

Die Erfindung betrifft einen Spülautomaten, insbesondere einen Desinfektionsautomaten, einen Endoskopspüler und/oder dergleichen, mit einem Spülbehälter, der einen Spülraum mit einer mittels einer Hubtür fluiddicht verschließbaren Spülraumöffnung aufweist, und mit einer Antriebseinrichtung für ein motorisches Verfahren der Hubtür, wobei die Antriebseinrichtung einen Elektromotor und einen damit zusammenwirkenden Zahnriemen aufweist, sowie mit einem die Hubtür gegenüber dem Zahnriemen kraftübertragend abstützenden Verbindungsstück.

DE 19514 303 offenbart einen Spülautomaten gemäß Oberbegriff des Anspruchs 1.

Spülautomaten der eingangs genannten Art sind aus dem Stand der Technik an sich bekannt. Es sei deshalb nur exemplarisch auf die EP 1 949 843 A1 verwiesen.

Ein Spülautomat der gattungsgemäßen Art verfügt über einen Spülbehälter, der in an sich bekannter Weise einen Spülraum zur Aufnahme von Spülgut bereitstellt. Der Spülraum ist über eine Spülraumöffnung des Spülbehälters zugänglich. Im bestimmungsgemäßen Verwendungsfall ist die Spülraumöffnung fluiddicht verschlossen, zu welchem Zweck gattungsgemäße Spülautomaten über eine Hubtür verfügen.

Typischerweise kommen Spülautomaten mit einer Hubtür im gewerblichen Bereich zur Anwendung. Spülautomaten für den Hausgebrauch verfügen in der Regel über eine verschwenkbar ausgebildete Drehtür.

Die Hubtür bei aus dem Stand der Technik vorbekannten Spülautomaten ist in der Regel motorisch verfahrbar ausgebildet, zu welchem Zweck eine entsprechende Antriebseinrichtung vorgesehen ist. Diese Antriebseinrichtung verfügt über einen Elektromotor sowie über einen damit zusammenwirkenden Zahnriemen. Dabei wird der Zahnriemen endlos umlaufend geführt und er ist mit der Hubtür kraftübertragend gekoppelt, und zwar mittels eines die Hubtür abstützenden Verbindungsstücks.

Bei einer Öffnungsbewegung wird der Zahnriemen vom Elektromotor in entsprechender Weise angetrieben. Das Verbindungsstück bewegt sich durch den Zahnriemen getragen in Höhenrichtung nach oben, so dass sich die darauf abstützende Hubtür öffnet, das heißt in ihrer Öffnungsstellung verfahren wird. Ein Schließvorgang geht in entgegengesetzter Richtung vonstatten, indem der Zahnriemen durch den Elektromotor angetrieben andersherum umläuft, wodurch das die Hubtür abstützende Verbindungsstück in Höhenrichtung nach unten wandert. In Folge dessen wird die sich gegenüber dem Verbindungsstück abstützende Hubtür nach unten bewegt.

Aus dem Stand der Technik vorbekannte Spülautomaten verfügen in aller Regel über zwei Zahnriemen der vorbeschriebenen Art, wobei mit Bezug auf eine Frontansicht des Spülautomaten ein erster Zahnriemen linksseitig der Hubtür und ein zweiter Zahnriemen rechtsseitig der Hubtür angeordnet sind.

Zum Schutz eines Anwenders ist die Hubtür für den Fall eines Reißens des oder der Zahnriemen gesichert ausgebildet. Dabei reagiert die aus dem Stand der Technik vorbekannte Sicherung auf das Reißen je eines der typischerweise vorgesehenen beiden Zahnriemen und löst eine mechanische Arretierung der Hubtür aus. Sollten tatsächlich beide Zahnriemen gleichzeitig oder kurz nacheinander reißen, wird so das ungebremste Abstürzen der Hubtür mit entsprechender Gefährdung für das Bedienpersonal verhindert.

Obgleich sich die aus dem Stand der Technik vorbekannte Absturzsicherung im alltäglichen Praxiseinsatz bewährt hat, besteht Verbesserungsbedarf. Insbesondere auch deshalb, weil es neben einem Reißen eines Zahnriemens auch zu anderen Versagungsarten und Schadensbildern kommen kann, die über die vorbekannte Absturzsicherung nicht mit abgedeckt sind. So kann es bei vorbekannten Spülautomaten beispielsweise bei einem Getriebebruch oder einem Bruch der Antriebswelle zu einem ungebremsten Abstürzen der Hubtür kommen, was ein erhebliches Gefahrenpotential für das Bedienpersonal darstellt. Die vorbekannten Sicherungseinrichtungen greifen in diesem Fall jedenfalls nicht ein, weil die Zahnriemen nach wie vor in Takt sind.

Es ist ausgehend vom Vorbeschriebenen deshalb die Aufgabe der Erfindung, einen Spülautomaten der eingangs genannten Art dahingehend weiterzuentwickeln, dass eine erweiterte Sicherheitseinrichtung vorgesehen ist, die nicht nur eine Absturzsicherung für den Fall eines Zahnriemenrisses bereitstellt.

Zur Lösung dieser Aufgabe wird mit der Erfindung ein Spülautomat der eingangs genannten Art vorgeschlagen, der zusätzlich die im kennzeichnenden Teil des Anspruchs 1 genannten Merkmale aufweist.

In an sich bekannter Weise stützt sich beim erfindungsgemäßen Spülautomat die Hubtür am Verbindungsstück ab, welches Verbindungsstück vom Zahnriemen getragen ist. Im Unterschied zum Stand der Technik ist mit der Erfindung ein Trennmittel vorgesehen, das zwischen Verbindungsstück und Hubtür angeordnet ist. Dieses Trennmittel sorgt bei einer Kraftlosstellung des Verbindungsstückes dafür, dass es zu einer Trennung von Verbindungsstück und Hubtür kommt. Dabei wirkt das Trennmittel mit einer Arretiereinrichtung für die Hubtür zusammen, die im Aktivierungsfall eine Arretierung der Hubtür gewährleistet, das heißt eine Lagesicherung der Hubtür.

Eine Kraftlosstellung des Verbindungsstückes kann auf unterschiedliche Weise zu Stande kommen. Dies kann zum Beispiel durch einen Riss des Zahnriemens geschehen. Aber auch dadurch, dass beispielsweise die Antriebswelle zwischen Getriebemotor und Zahnriemenräder bricht, das zwischen Motor und Zahnriemen vorgesehene Getriebe einen Defekt aufweist und/oder die Selbsthemmung des Antriebsstrangs nachlässt. In all diesen Fällen kann die vom Verbindungsstück auf die Hubtür übertragene Kraft zur Stützung der Hubtür nicht aufrecht erhalten werden, so dass das Verbindungsstück im Ergebnis kraftlos gestellt wird, so dass es in der vorbeschriebenen Weise aufgrund des zwischen Verbindungsstück und Hubtür angeordneten Trennmittels zu einer Trennung von Verbindungsstück und Hubtür kommt.

Das erfindungsgemäß vorgesehene Trennmittel kommt auch bei einer Trennung der Drehmomentverbindung zwischen Getriebemotor und Antriebswelle, bei einer Depositionierung des Antriebsmotors und/oder bei einem Versagen der tragenden Verbindung zwischen Hubtür und Zahnriemen zum Einsatz. Mit der erfindungsgemäßen Ausgestaltung wird insofern eine Sicherung vorgeschlagen, die im Unterschied zum Stand der Technik auf eine Vielzahl von möglichen Versagungsarten anspricht und sicherstellt, dass in jedem Fall eine Lagesicherung der Hubtür, das heißt eine Arretierung derselben über eine Arretiereinrichtung stattfindet, nicht zuletzt zum Schutz des Bedienpersonals.

Bei dem Trennmittel nach der Erfindung kann es sich beispielsweise um eine Druckfeder handeln. Im bestimmungsgemäßen Normalzustand ist die Druckfeder zwischen dem Verbindungsstück und der Hubtür in zusammengestauchten Zustand eingeklemmt. Die Gewichtskraft der Hubtür ist insofern ausreichend. Kommt es im Versagensfall zu einer Kraftlosstellung des Verbindungsstückes, so wird über das Auffedern der Druckfedern ein Trennen von Verbindungsstück und Hubtür dadurch erreicht, dass die Druckfeder in Folge ihres Auffederns das Verbindungsstück relativ zur Hubtür von dieser wegdrückt.

Die Arretiereinrichtung nach der Erfindung verfügt über einen Arretierhebel einerseits und ein damit zusammenwirkendes Widerlager andererseits, zu welchem Zweck das Widerlager mit Aussparungen ausgerüstet ist, in die der Arretierhebel mit einem widerlagerseitigen Fortsatz eingreifen kann.

Der Arretierhebel ist verschwenkbar an der Hubtür angeordnet und wirkt mit dem Verbindungsstück und/oder dem Trennmittel derart zusammen, dass bei einer Kraftlosstellung des Verbindungsstücks ein Verschwenken des Arretierhebels stattfindet, in Folge dessen es zu einem Einrasten des vom Arretierhebel widerlagerseitig bereitgestellten Fortsatzes in eine vom Widerlager bereitgestellte Aussparung kommt. In der eingerasteten Stellung ist die Hubtürlage gesichert, das heißt gegenüber dem Widerlager arretiert.

Im Falle der Ausgestaltung des Trennmittels als Druckfeder ist bevorzugter Weise vorgesehen, dass der Arretierhebel hubtürseitig eine die Druckfeder einendseitig aufnehmende Vertiefung aufweist. Die Druckfeder stützt sich gemäß dieser Ausführungsform unter Zwischenschaltung des Arretierhebels gegenüber dem Verbindungsstück ab, wobei die Druckfeder hebelseitig in eine dafür vorgesehene Vertiefung eingreift. Kommt es in Folge eines Versagensfalls zu einer kraftlosen Stellung des Verbindungsstücks, so federt die Druckfeder in der schon vorbeschriebenen Weise auf, was aufgrund der Abstützung der Druckfeder gegenüber dem Arretierhebel zu einer sofortigen Verschwenkung desselben mit der Konsequenz führt, dass es zu einer Lagesicherung der Hubtür durch Einrasten des Arretierhebels in eine vom Widerlager bereitgestellte Aussparung kommt.

Neben dem schon vorbeschriebenen Spülautomaten wird mit der Erfindung zur Lösung der eingangs genannten Aufgabe des Weiteren vorgeschlagen eine Arretiereinrichtung für eine Hubtür eines Spülautomatens, mit einem Arretierhebel, der einendseitig mit einer an der Hubtür angeordneten Druckfeder und anderendseitig mit einem mit Aussparungen ausgerüsteten Widerlager zusammenwirkt, wobei der Arretierhebel verschwenkbar an der Hubtür angeordnet ist. Eine in solcher Weise ausgestaltete Arretiereinrichtung erbringt die schon vorbeschriebenen Vorteile.

Weitere Vorteile und Merkmale der Erfindung ergeben sich aus der nachfolgenden Beschreibung anhand der Figuren. Dabei zeigen
- Figur 1: in einer schematischen Seitendarstellung eine Arretiereinrichtung nach der Erfindung gemäß einer ersten Stellung und
- Figur 2: in einer schematischen Seitendarstellung eine Arretiereinrichtung nach der Erfindung gemäß einer zweiten Stellung.

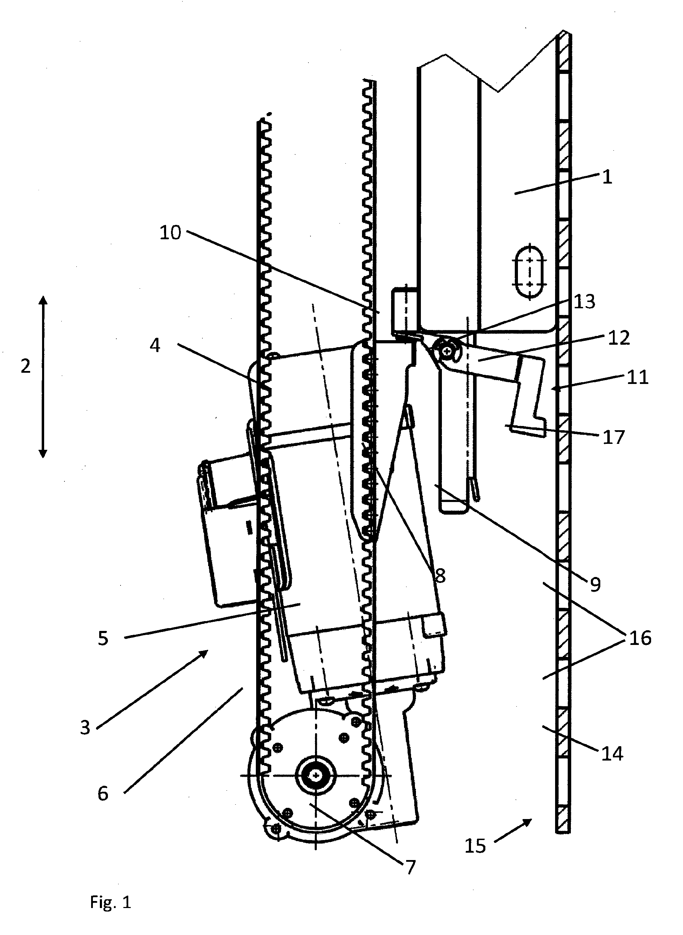

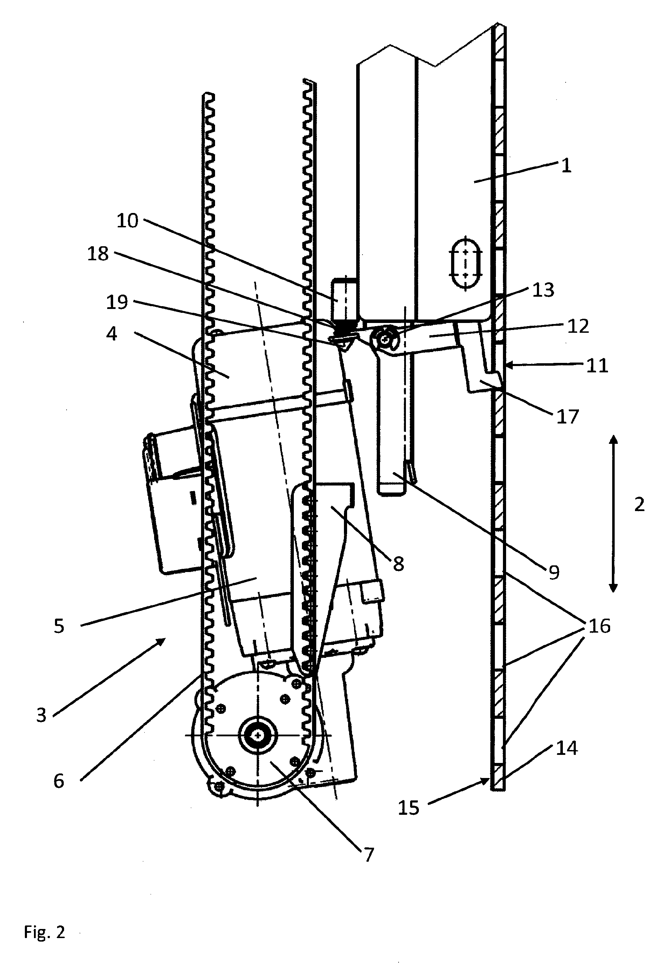

Die Figuren 1 und 2 lassen jeweils in schematischer Darstellung eine erfindungsgemäße Arretiereinrichtung 11 erkennen, wobei Figur 1 die unausgelöste Stellung und Figur 2 die ausgelöste Stellung der Arretiereinrichtung 11 zeigt.

Die Figuren 1 und 2 lassen ausschnittsweise eine Hubtür 1 eines der besseren Übersicht wegen ansonsten nicht weiter dargestellten Spülautomatens erkennen. Die Hubtür 1 dient dem fluiddichten Verschließen einer Spülraumöffnung. Die Hubtür 1 ist in Höhenrichtung 2 verfahrbar angeordnet. Die Hubtür 1 ist im gezeigten Ausführungsbeispiel motorisch verfahrbar ausgebildet, zu welchem Zweck eine Antriebseinrichtung 3 vorgesehen ist. Diese Antriebseinrichtung 3 verfügt über einen Elektromotor 4, ein Getriebe 5 und einen Zahnriemen 6. Dabei erfolgt ein Antrieb des Zahnriemens 6 über die Zahnriemenscheibe 7, die mit dem Getriebe 5 gekoppelt ist.

Der Zahnriemen 6 trägt ein Verbindungsstück 8. Dieses Verbindungsstück 8 stützt die Hubtür 1 ab.

Im Öffnungsfall erfolgt über den Elektromotor 4, das Getriebe 5 und die Zahnriemenscheibe 7 ein Antrieb des Zahnriemens 6 in der Weise, dass er entgegen des Uhrzeigersinns verfahren wird. In Folge dessen bewegt sich das Verbindungsstück 8 und damit auch die sich darauf abstützende Hubtür 1 in Höhenrichtung 2 nach oben. Im umgekehrten Fall, wenn der Zahnriemen 6 mit dem Uhrzeigersinn laufend angetrieben wird, bewegt sich das Verbindungsstück 8 mit Bezug auf die Zeichnungsebene nach den Figuren 1 und 2 nach unten, was zu einer Verfahrbewegung der Hubtür 1 ebenfalls nach unten führt.

Die Darstellung nach den Figuren lässt einen Zahnriemen 7 und ein Verbindungsstück 8 erkennen. In der Regel sind mit Bezug auf eine Frontansicht der Hubtür 1 sowohl linksseitig als auch rechtsseitig jeweils ein Zahnriemen 6 und ein entsprechendes Verbindungsstück 8 vorgesehen, so dass die Hubtür 1 beidseitig abgestützt ist.

Erfindungsgemäß ist zwischen dem Verbindungsstück 8 und der Hubtür 1 ein Trennmittel angeordnet, das im gezeigten Ausführungsbeispiel eine Druckfeder 19 ist. Diese Druckfeder ist in einem an der Hubtür 1 angeordneten Gehäuse 10 untergebracht.

Es ist erfindungsgemäß des Weiteren eine Arretiereinrichtung 11 vorgesehen, die mit der Druckfeder 19 zusammenwirkt. Diese Arretiereinrichtung 11 verfügt über einen Arretierhebel 12 und ein Widerlager 14.

Der Arretierhebel 12 ist von der Hubtür 1 getragen, zu welchem Zweck die Hubtür 1 einen Arm 9 bereitstellt, die den Arretierhebel 12 um einen Drehpunkt 13 verschwenkbeweglich aufnimmt.

Das Widerlager 14 ist durch einen Rahmen 15 gebildet, der Aussparungen 16 bereit stellt. Diese Aussparungen 16 wirken mit dem Arretierhebel 12 zusammen, zu welchem Zweck der Arretierhebel 12 widerlagerseitig einen Fortsatz 17 trägt, der in die vom Widerlager 14 bereitgestellten Aussparungen eintauchen kann.

Der Arretierhebel 12 verfügt ferner über eine die Druckfeder 19 einendseitig aufnehmende Vertiefung 18, wie sich insbesondere aus der Darstellung nach Figur 2 ergibt.

Die Funktionsweise der erfindungsgemäßen Arretiereinrichtung 11 ergibt sich wie folgt:

Im störungsfreien Betriebszustand, wie er in Figur 1 dargestellt ist, stützt sich die Hubtür 1 gegenüber dem Verbindungsstück 8 ab, und zwar unter Zwischenschaltung des drehbar an der Hubtür 1 gelagerten Arretierhebels 12. In der schon vorbeschriebenen Weise kann ein Öffnen und Schließen der Hubtür 1 durch ein Verfahren des Verbindungsstückes 8, das heißt ein Betreiben der Antriebseinrichtung 3 bewirkt werden.

Kommt es zu einem Störungsfall, in Folge dessen das Verbindungsstück 8 kraftlos gestellt wird, sei es beispielsweise durch einen Zahnriemenriss, einen Motor- oder Getriebeschaden, einen Bruch der die Zahnriemenscheibe 7 tragenden Antriebswelle und/oder dergleichen, so federt die vom Gehäuse 10 gehaltene Druckfeder 19 aus. In Folge dieser Ausfederung kommt es zu einer Verschwenkbewegung des Arretierhebels 12 um den Drehpunkt 13 herum, in Folge dessen der widerlagerseitig getragene Fortsatz 17 des Arretierhebels 12 in eine der vom Widerlager 14 bereitgestellten Aussparungen 16 eintaucht. Diese gesicherte Position ist in Figur 2 dargestellt.

In der gesicherten Position, wenn also der Arretierhebel 12 mit seinem Fortsatz 17 in eine vom Widerlager 14 bereitgestellte Aussparung 16 eingreift, ist eine Lagefixierung und damit Sicherung der Hubtür 1 erreicht. Ein ungebremster Absturz der Hubtür 1 ist jedenfalls in dieser arretierten Stellung nicht mehr möglich.

### Bezugszeichenliste

- 1: Hubtür
- 2: Höhenrichtung
- 3: Antriebseinrichtung
- 4: Elektromotor
- 5: Getriebe
- 6: Zahnriemen
- 7: Zahnriemenscheibe
- 8: Verbindungsstück
- 9: Arm
- 10: Gehäuse
- 11: Arretiereinrichtung
- 12: Arretierhebel
- 13: Drehpunkt
- 14: Widerlager
- 15: Rahmen
- 16: Aussparung
- 17: Fortsatz
- 18: Vertiefung
- 19: Druckfeder

## Patentansprüche

1. Spülautomat, insbesondere Desinfektionsautomat, Endoskopspüler, mit einem Spülbehälter, der einen Spülraum mit einer mittels einer Hubtür (1) fluiddicht verschließbaren Spülraumöffnung aufweist, und mit einer Antriebseinrichtung (3) für ein motorisches Verfahren der Hubtür (1), wobei die Antriebseinrichtung (3) einen Elektromotor (4) und einen damit zusammenwirkenden Zahnriemen (6) aufweist, sowie mit einem die Hubtür (1) gegenüber dem Zahnriemen (6) kraftübertragend abstützenden Verbindungsstück (8),
**gekennzeichnet durch**
ein zwischen dem Verbindungsstück (8) und der Hubtür (1) angeordnetes Trennmittel (19), welches bei einer Kraftlosstellung des Verbindungsstücks (8) dafür sorgt, dass es zu einer Trennung von Verbindungsstück (8) und Hubtür (1) kommt,
und eine mit dem Trennmittel zusammenwirkende Arretiereinrichtung (11) für die Hubtür (1).

2. Spülautomat nach Anspruch 1,
**dadurch gekennzeichnet,**
**dass** das Trennmittel eine Druckfeder (19) ist.

3. Spülautomat nach Anspruch 2,
**dadurch gekennzeichnet,**
**dass** die Druckfeder (19) verbindungsstückseitig an der Hubtür (1) angeordnet ist.

4. Spülautomat nach einem der vorhergehenden Ansprüche,
**dadurch gekennzeichnet,**
**dass** die Arretiereinrichtung (11) einen Arretierhebel (12) und ein mit Aussparungen (16) ausgerüstetes Widerlager (14) aufweist.

5. Spülautomat nach Anspruch 4,
**dadurch gekennzeichnet,**
**dass** der Arretierhebel (12) an der Hubtür (1) angeordnet ist.

6. Spülautomat nach Anspruch 5,
**dadurch gekennzeichnet,**
**dass** der Arretierhebel (12) verschwenkbar an der Hubtür (1) angeordnet ist.

7. Spülautomat nach einem der vorhergehenden Ansprüche 4 bis 6,
**dadurch gekennzeichnet,**
**dass** das Widerlager (14) Teil eines die Spülraumöffnung umgebenden Rahmens (15) ist.

8. Spülautomat nach einem der vorhergehenden Ansprüche 4 bis 7,
**dadurch gekennzeichnet,**
**dass** der Arretierhebel (12) widerlagerseitig einen Fortsatz (17) aufweist, der korrespondierend zu den Aussparungen (16) des Widerlagers (14) ausgebildet ist.

9. Spülautomat nach einem der vorhergehenden Ansprüche 4 bis 8,
**dadurch gekennzeichnet,**
**dass** der Arretierhebel (12) hubtürseitig eine die Druckfeder (19) einendseitig aufnehmende Vertiefung (18) aufweist.

## Claims

1. Automatic rinsing machine, in particular a disinfection machine, endoscope washer, comprising a washing container, which has a rinsing chamber having a rinsing chamber opening which can be closed so as to be impermeable to fluids by means of a lift door (1), and comprising a drive device (3) for motorised displacement of the lift door (1), the drive device (3) having an electric motor (4) and a toothed belt (6) which interacts with said motor and also with a connecting piece (8) which supports the lift door (1) by transmitting force relative to the toothed belt (6),
**characterised by**
a separating means (19) arranged between the connecting piece (8) and the lift door (1), which separating means ensures that the connecting piece (8) and the lift door (1) separate when the connecting piece (8) is without force, and a locking apparatus (11) for the lift door (1), which apparatus interacts with the separating means.

2. Automatic rinsing machine according to claim 1,
**characterised in that**
the separating means is a compression spring (19).

3. Automatic rinsing machine according to claim 2,
**characterised in that**
the compression spring (19) is arranged on the lift door (1) on the connection-piece side.

4. Automatic rinsing machine according to any of the preceding claims,
**characterised in that**
the locking apparatus (11) comprises a locking lever (12) and an abutment (14) equipped with recesses (16).

5. Automatic rinsing machine according to claim 4,
**characterised in that**
the locking lever (12) is arranged on the lift door (1).

6. Automatic rinsing machine according to claim 5,
**characterised in that**
the locking lever (12) is arranged pivotally on the lift door (1).

7. Automatic rinsing machine according to any of the preceding claims 4 to 6,
**characterised in that**
the abutment (14) is part of a frame (15) surrounding the rinsing chamber opening.

8. Automatic rinsing machine according to any of the preceding claims 4 to 7,
**characterised in that**
the locking lever (12) comprises an extension (17) on the abutment side, which is formed to correspond with the recesses (16) of the abutment (14).

9. Automatic rinsing machine according to any of the preceding claims 4 to 8,
**characterised in that**
the locking lever (12) comprises a depression (18) on the lift-door side for receiving the compression spring (19) at one end.

## Revendications

1. Automate de rinçage, en particulier automate de désinfection, dispositif de rinçage d'endoscope, avec une cuve de rinçage, qui présente une chambre de rinçage avec un orifice de chambre de rinçage pouvant être fermé de façon étanche aux fluides au moyen d'une porte guillotine (1), et avec un dispositif d'entraînement (3) pour un déplacement motorisé de la porte guillotine (1), le dispositif d'entraînement (3) présentant un moteur électrique (4) et une courroie dentée (6) qui coopère avec ce dernier, ainsi qu'avec une pièce de raccordement (8) soutenant la porte guillotine (1) en assurant la transmission de force par rapport à la courroie dentée (6),
**caractérisé par**
un moyen de séparation (19) qui est disposé entre la pièce de raccordement (8) et la porte guillotine (1) et qui assure, quand la pièce de raccordement (8) est privée de force, une séparation entre la pièce de raccordement (8) et la porte guillotine (1), et par un dispositif de blocage (11) pour la porte guillotine (1) qui coopère avec le moyen de séparation.

2. Automate de rinçage selon la revendication 1,
**caractérisé en ce que**
le moyen de séparation est un ressort de compression (19).

3. Automate de rinçage selon la revendication 2,
**caractérisé en ce que** le ressort de compression (19) est disposé sur la porte guillotine (1) côté pièce de raccordement.

4. Automate de rinçage selon une des revendications précédentes,
**caractérisé en ce que**
le dispositif de blocage (11) présente un levier de blocage (12) et une butée (14) équipée d'évidements (16).

5. Automate de rinçage selon la revendication 4,
**caractérisé en ce que**
le levier de blocage (12) est disposé sur la porte guillotine (1).

6. Automate de rinçage selon la revendication 5,
**caractérisé en ce que**
le levier de blocage (12) est disposé de façon pivotante sur la porte guillotine (1).

7. Automate de rinçage selon une des revendications précédentes 4 à 6,
**caractérisé en ce que**
la butée (14) fait partie d'un cadre (15) entourant l'orifice de chambre de rinçage.

8. Automate de rinçage selon une des revendications précédentes 4 à 7,
**caractérisé en ce que**
le levier de blocage (12) présente, côté butée, un prolongement (17) qui est constitué d'une façon qui correspond aux évidements (16) de la butée (14).

9. Automate de rinçage selon une des revendications précédentes 4 à 8,
**caractérisé en ce que**
le levier de blocage (12) présente, côté porte guillotine, un creux (18) logeant le ressort de compression (19) d'un côté d'une extrémité.
